# EUROPEAN PATENT APPLICATION

(11) **EP 1 614 427 A1**
(43) Date of publication of application: **11.01.2006**
(21) Application number: 04721813.6
(22) Date of filing: 19.03.2004
(51) Int. Cl.: A61K 39/106, A61K 9/28

(54) **INACTIVATED VIBRIO CHOLERAE VACCINE IN TABLET FORM**

(30) Priority: 20.03.2003 CU 6103
(71) Applicant: Instituto Finlay, Centro de Investigacion-Produccion de vacunas y sueros, La Coronela, La Lisa, Ciudad Habana 11600 (CU)
(72) Inventor: TALAVERA CORONEL, Arturo, Ciudad de la Habana, 10400 (CU); ANO LOPEZ, Gemma, Ciudad de la Habana, 10700 (CU); CASTANO FERN NDEZ, Jorge, Luis, Ciudad de la Habana, 12500 (CU); URRIBARRI HERN NDEZ, Evangelina, CIUDAD DE LA HABANA, 11600 (CU); PINO NAVARRO, Yadira, CIUDAD DE LA HABANA, 10400 (CU); GARCIA SANCHEZ, Hilda, Maria, CIUDAD DE LA HABANA, 11600 (CU); BALMASEDA PEREZ, Tania, CIUDAD DE LA HABANA, 10400 (CU); CEDR MARRERO, Bárbara, CIUDAD DE LA HABANA, 11600 (CU); GARCIA IMIA, Luis, Playa, CIUDAD DE LA HABANA, 11600 (CU); P REZ QUINOY, José, Luis, CIUDAD DE LA HABANA, 11600 (CU); TORRES SUAREZ, Caridad, CIUDAD DE LA HABANA, 11600 (CU)
(74) Representative: Gil-Vega, Victor
(86) International application number: PCT/CU2004/000005
(87) International publication number: WO 2004/082711

(57) **Abstract**

The present invention has applications in the field of vaccinology, specifically as an orally administered vaccine for the cholera infection. The aim is to develop a vaccine for oral administration in tablets derived from whole, inactivated cells of *Vibrio cholera,* and to disclose a method for obtaining this vaccine preparation.

## Description

The present invention has applications in the field of vaccinology, specifically as an orally administered vaccine for the cholera infection.

The invention discloses a new vaccine formula and its therapeutic applications. More specifically, the invention presented consists of an inactivated vaccine formula, reactive to *Vibrio cholera,* that can be orally administered in tablets.

Cholera is an infectious disease whose most notable symptom is an acute diarrhea characterized by sudden and profuse discharges of liquid feces. The diarrhea can be quite severe and can even cause death within a period of 5 hours following the onset of symptoms if the patient does not receive adequate medical attention. It is acquired orally, from contaminated foods or liquids. Seven epidemics -all resulting in elevated indices of morbidity and mortality- have been reported to the present day. The infection is most common among populations inhabiting underdeveloped areas or regions affected by natural disasters, wars or other destructive events, which bring about poor sanitary conditions. Travelers with destinations that are thus affected are also susceptible to the infection (CDC/NCID. Organizacion Panamericana de la Salud. Metodos de laboratorio para el diagnóstico de *Vibrio cholerae.* En: Programa especial de publicaciones. Ed. Washington, D.C., Estados Unidos 1994).

Though treatment with antibiotics and hydrating salts is effective, thorough and consistent medical attention is needed to respond to the rapid effects of the disease. On the other hand, it has been shown that the infection induces a specific immune protection associated to the presence of vibriocidal antibodies in the serum, as indicator of people's protection (Glass, R.I., Svennerholm, A.M., Stoll, B.J.,Khan, M.R., Hossain, K.M., Huq, M.I., Holmgren, J. 1983. Protection against cholera in breast-fed children by antibodies in breast milk. N. Engl. J. Med. 308: 1389-1392 and Jertborn, M., Svennerholm, A. M., Holmgren, J. 1993. Evaluation of different immunization schedules for oral B subunit vaccine in Swedish volunteers. Vaccine. 10: 130-2). This has led to the development of different classes of cholera vaccines, derived from either whole inactivated cells or from live attenuated cells.

Parenterally administered vaccines derived from whole cells inactivated with phenol were removed from the market because of their short-lived (from 3 to 6 months) and low level (30 to 60%) of protection, as well as their high levels of reactogenesis, leading to erythema, localised pains, induration, fever and cephalia (OMS. 1991. Reunión sobre la vacuna contra el cólera. Informe final. Washington, D.C.).

An alternative is found in whole cells inactivated with bile (the Bilivaccines). This orally administered vaccine offers a moderate level of protection and has not been industrially developed (Svennerholm, A. M. and Holmgren, J. 1986. Oral combined B subunit-whole cell cholera vaccine. In J. Holmgren A. Lindberg and R. Mollby (ed). Development of vaccines and drugs against diarrhea. 11^{th} Novel Conference, Stockholm, 1985. Student litteratur, Lund, Sweden. p.33-43).

It has been shown that whole cells inactivated with heat and formaldehyde, combined with the B-subunit of the cholera toxin (B-WC) offer moderate levels of protection (that of 60%) three years after being administered (Holmgren, J., Svennerholm, A., M., Lonroth, I., Fall-Persson, M., Markham, B. and Lundback, H. 1977. Development of improved cholera vaccine based on subunit toxoid. Nature 269:602-604); but production of this vaccine is extremely expensive and complex, because of the need to obtain and purify the B-subunit of the cholera toxin (Tayot, J. L., Holmgren, J., Svennerholm, L., Lindblad, M., Tardy, M. 1981. Receptor-specific large scale purification of cholera toxin on silica beads derivatized with lyso-GMI gaglioside. Eur. J. Biochem. 113:249-258).

A variant of the above mentioned alternative, employing the B-subunit of the cholera toxin obtained through the recombinant method (rB-WC), proves reliable but still offers a mere 60% level of protection three years after being administered and it involves a bothersome preparation process. The vaccine is prepared in two multi-layered envelopes, one containing a mixture of salts in powder form and the other containing the freeze-dried strain of *Vibrio cholerae.* At the moment of administration, the first must be re-suspended in water and the second in a solution of salts and water; the solution containing the salts must then be orally administered, followed by the suspension containing the strain, administered in the same fashion 15 minutes later (Sánchez, J. L., Holmgren, J. 1989. Recombinant system for overexpression of cholera toxin B Subunit in *Vibrio cholerae* as basis for vaccine development. Proc. Natl. Acad. Sci. USA 86:481-485).

Another state-of-the-art vaccine derived from whole inactivated cells is similar to this last one, substituting the *Vibrio cholerae* Cairo 50 strain (Classic, Ogawa) with a 569B strain (El Tor, Inaba). It offers an acceptable level of protection (that of 66%) up to 8 to 10 months after being administered. It is a mixture of strains of different serotypes and bio-types of *Vibrio cholerae ,* and it is prepared in a bottle containing the suspended strain and an envelope containing a mixture of salts (Trach, D. D., Clemens, J.D., Ke, N.T., Thuy, H.T.,Son, N.D., Canh, D.G., Hang, P.V.D, Rao, M.R. 1997. Field trial of a locally produced, killed, oral cholera vaccine in Vietnam. Lancet. 349:231-235) As the above mentioned vaccine, its application requires that the mixture of salts be dissolved in water and orally administered, and that the contents of the bottle containing the cell strain be administered 15 minutes later, making for a slow and cautious procedure that requires time as well as qualified personnel, and the process all the more costly.

The CVD103 HgR vaccine, based on the CVD103 strain, supplemented by a mercury-resistant gene that is additionally inserted into the strain in order to differentiate the epidemic strain, involves a safe method of administration and resulted in good levels of protection in the trials conducted on volunteers, though it must be administered in high doses to obtain a substantial immunogenic response (OMS. 1991. Reunion sobre la vacuna contra el cólera. Informe final. Washington, D.C. and Holmgren, J., and Svennerholm, A. M. 1999. Vaccines against Diarrheal Diseases. In Perlmann, P. and Wigzell, H. [ed]. Vaccines. Springer-Verlag Berlin Heidelberg New York. p:290-328). The production of this strain requires entirely closed systems ( Cryz, S.J. and Gluck, R., 1997. Large-Scale production of live attenuated bacterial and viral vaccines. In New Generation Vaccine. Second Edition. Ed. Levine, M.M., Woodrow, G.C.,Kaper, J.B. and Cobon, G.S. Library of Congress. Cataloguing in Publication Data. New York. P: 1153-1163) and it is prepared in the same fashion as the previously mentioned vaccine, presenting the same difficulties at the time of administration.

The live attenuated vaccine based on the 638 strain, derived from the C7258 epidemic strain, which was treated to eliminate the CTXφ virulent phage and include the vaccine marker celA in the hap gene that codifies protease hemaglutinins, proved reliable at the time of application and resulted in an adequate immunological response (Benitez, J.A., García L., Silva A., García, H., Fando, R., Cedré, B., Pérez, A., Campos, J., Rodríguez, B., Pérez, J.L., Balmaseda, T., Pérez, O., Ramirez, M., Ledón, T., Diaz, M., Bravo, L. and Sierra, G. 1999. Preliminary assessment of the safety and immunogenicity of a new CTXφ- negative, haemaglutinin/protease-defective El Tor strain as a cholera vaccine candidate. Infect. Immun. 67: 539-545). Due to a preparation process similar to those mentioned above, however, it presents all of difficulties associated with the previously considered vaccines.

The vaccines based on the attenuation of *Vibrio cholerae* strains, making use of such strains as Peru-3, Bah-3 and Bang-3, though offering immunogenic responses, proved unacceptably reactogenic (Taylor, D.N, Killen, K.P., Hack, D.C>, Kenner, J.R., Coster, T.S., Beatle, D.T., Ezzell, J., Hyman, T., Tropa, A., Sjogrem, M.H., Friedlander, A., Mekalanos, J.J., Sadoff, J.C. 1994. Development of a live, oral, attenuated vaccine against El Tor cholera. J. Infect. Dis. 170:1518-1523).

The vaccine employing the Peru 15 strain, based on the Peru-3 strain, has a safe application method and yielded good levels of protection in trials conducted on volunteers (Kener, J.R., Coster, T.S., Taylor, D.N., Troffa, A.F., Barrera-Oro, M., Hyman, T., Adams, J.M., Beattie, D.T., Killeen, K.P., Spriggs, D.R., Mekalanos, J.J., Sadoff, J.C. 1995. Peru-15, an improved and attenuated oral vaccine candidate for *Vibrio cholerae* O1. J. Infect. Dis. 72:1126-1129). No clinical studies have been carried out of yet, however, and the vaccine presents the same difficulties as those mentioned above.

The present invention consists of a new vaccine formula that includes: whole inactivated cells of *Vibrio cholerae,* agglutinants, lubricants, coating, filling and disintegrating substances used for its final preparation in the form of tablets, which maintains its various components in a stable condition and prevents them from interfering with each other.

It was surprising to find that the final product yielded and antigenic and immunogenic effect similar to that offered by the inactivated cells that made up the active complex of the formula.

The present invention provides a vaccine composed of inactivated cells of the *Vibrio cholerae* strain belonging to the serum group 0139, or to serum group O1, and to the classic, El Tor, Ogawa or Inaba bio and serum-types, employing wild or attenuated strains.

The vaccine formula here presented contains inactivated cells of *Vibrio cholerae,* preferably between 10⁹ and 10¹¹ inactivated bacteria per tablet. It can be made to contain the agglutinants: povidone, gelatin, carboxymethylcellulose, which must be found at a concentration of 1 to 5% in relation to the tablet's total mass; the lubricants: sodium carboxymethylstarch, magnesium stearate, colloidal silicon dioxide and talc, to be used at a concentration between 0.25 and 1.5% in relation to the tablet's total mass; the coating substances: cellulose acetophthalate, cellulose diethylphthalate, lacquer at a concentration of 10%, and titanium dioxide, to be used at a 1 to 2% concentration in relation to the tablet's total mass; the filling substances: lactose and cornstarch, which must be found at a concentration of 65 to 80% in relation to the total mass of the tablet, and disintegrating substances: sodium croscaramelose, cornstarch and micro-crystalline cellulose, to be used at a concentration between 5 and 15% in relation to the tablet's total mass.

To this day, there is no precedent in state-of-the-art vaccinological methods to the present tablet formula. It should also be pointed out that the present formula is the first to combine synthetic compounds with biological material, particularly cells, in a preparation that resists the chemical and physical stresses characteristic of the technology in use without thereby affecting the main immunological properties of its active complex.

The object of the present invention shall be described through the following examples:
**Example 1**: Study on the possible interferences of the excipients found in the tablets during an ELISA test used in the evaluation of the LPS of *Vibrio cholerae.*
   Inactivated cells and mixtures of these including each of the different excipients, as well as separate solutions of excipients, were studied for this example. The inactivated cells were employed as a positive control group. The excipients used were: sodium carboxymethylstarch, sodium croscaramelose, povidone, talc, magnesium stearate, titanium dioxide, colloidal silicon dioxide, cornstarch and lactose.
   The ELISA technique consisted of the following steps:
   1. Cover the polystyrene plaques (Maxisorp, Nunc, Denmark) with 100 µL/hole of a cell mixture - excipients dissolved in a PBS. Incubate overnight at 4°C in a wet chamber.
   2. Wash the plaques with 0.05% Tween 20 in water between each of the steps of the immunoenzymatic trial.
   3. Block the plaques with 150 µL/hole of skim milk at a 1% concentration in a PBS. Incubate for 1 hour at 37°C in a wet chamber.
   4. Add 100 µL/hole of an anti-LPS monoclonal antibody
   5. Add 100 µL/hole of an anti-IgG antibody extracted from a mouse, conjugated with type VI horseradish peroxidase (Sigma Chemical Co. Saint Louis MO) diluted in a PBS with 0.05% Tween 20 and 1% skim milk. Incubate for 2 hours at room temperature in a wet chamber.
   6. Develop the reaction with 100 µL/hole of an o-phenylenediamine solution (Sigma Chemical Co. Saint Louis MO) at a concentration of 0.4 mg/mL in a citrate buffer solution at 0.1 M pH 4.5, containing 0.01 % of H₂0₂.
   7. Stop the reaction after 30 minutes following the application of a substrate with 50 µL/hole of a solution of 2.5 M of H₂SO₄, and read the plaque at a wavelength of 492 nm in a micro-plaque reader.

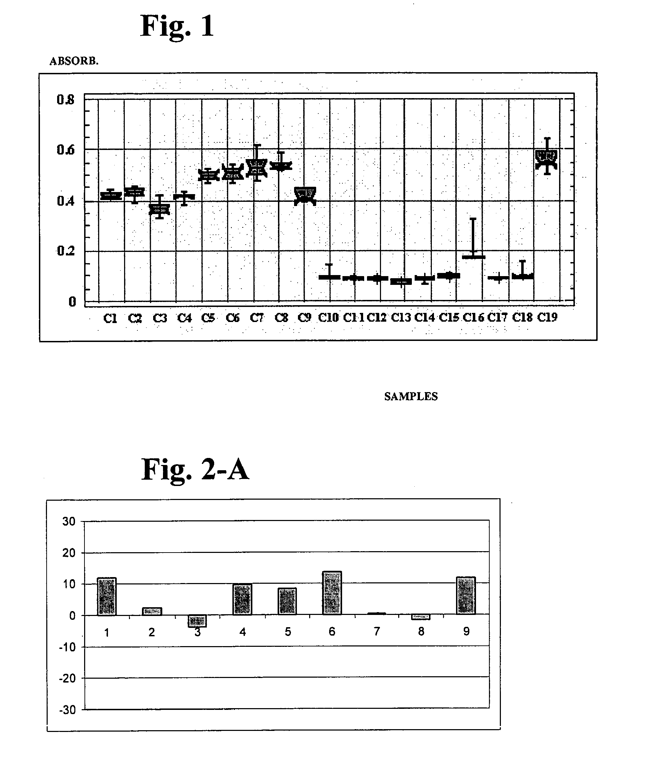

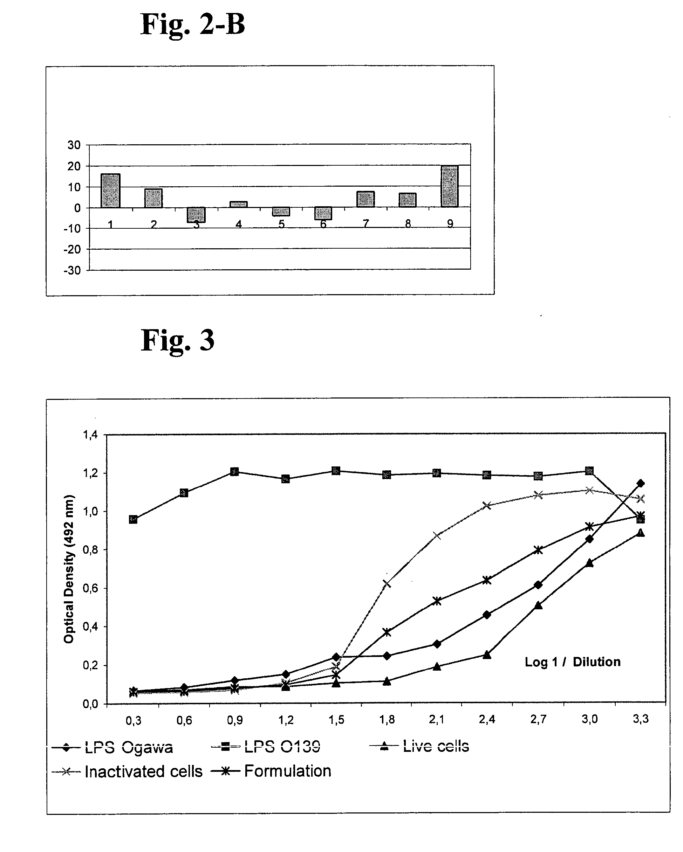

   The results demonstrated that no interferences occur between the excipients employed and the components of the analytic process, as shown in Figure 1, where the mixtures of the excipients with the inactivated cells present similar values to those of the inactivated cells alone, while the unmixed excipients offered significantly lower values.
**Example 2**: Determination of the possible interferences between the excipients and the vaccine's active complex.
   The antigenicity of the mixtures containing a suspension of inactivated *Vibrio cholerae* cells and the different excipients used was determined using the ELISA technique (as described in Example 1), dividing the mixtures into two, equal parts and incubating one at 4°C and the other at 28°C. The study was carried out at time 0 and after 30 days of incubation. The excipients used in this example were: lactose, starch, sodium carboxymethylstarch, croscaramelose, povidone, talc, magnesium stearate and titanium dioxide. The results indicated that the antigenicity of the lipopolysaccharide of the inactivated cells was not affected by the presence of the different excipients. The ELISA values were maintained throughout the 30 days of incubation, both in the trial conducted at 4°C and the one conducted at 28°C. (Fig. 2)
**Example 3**: Antigenicity of the vaccine.
   The vaccine's antigenicity was determined through an ELISA inhibition test aimed at detecting the lipopolysaccharide (LPS) of *Vibrio cholerae* in the tablets.
   Solutions of the tablets, LPS Ogawa of *V. cholerae,* LPS O139 of *V. cholerae,* a suspension of live *V. cholerae* cells of the El Tor and Ogawa strain, and a suspension of inactivated cells were used as samples for the test.
   The ELISA test consisted of the following steps:
   Cover the polystyrene plaques (Maxisorp, Nunc, Denmark) with 100 µL/hole of LPS Ogawa of *V. cholerae* at a concentration of 25 µg/mL, dissolved in a phosphate buffer solution (PBS). Incubate overnight at 4°C in a wet chamber.
   Wash the plaques with 0.05% Tween-20 in water between each of the steps of the immunoenzymatic trial.
   Block the plaques with 150 µL/hole of skim milk at a concentration of 1% in a PBS. Incubate for 1 hour at 37°C in a wet chamber.
   Add 100 uL of the samples previously incubated for 1 hour at room temperature in solutions with a concentration ratio of 1:2 for the samples and anti-LPS Ogawa monoclonal antibodies, respectively, dissolved in a PBS 0.05% Tween 20 to a final concentration of 25 ug/ml. Incubate for 2 hours at room temperature in a wet chamber.
   Add 100 ul/hole of species-specific anti-IgG antibody conjugated with type VI horseradish peroxidase (Sigma Chemical Co. Saint Louis MO) diluted in a PBS 0.05% Tween 20, with a skim milk at a concentration of 1%. Incubate for 2 hours at room temperature in a wet chamber.
   Develop the reaction with 100 ul/hole of a solution of o-phenylenediamine (Sigma Chemical Co. Saint Louis) to a final concentration of 0.4 mg/mL in a citrate buffer at 0.1 M pH 4.5 containing 0.01% of H₂O₂.
   Stop the reaction after 30 minutes. After adding the substrate with 50 uL/hole of a solution of 2.5 M of H₂SO₄, read the plaque at a wavelength of 492 nm in a Titerkek Multiskan Plus micro-plaque reader (Flow Laboratories, USA).
   The results showed a high level of inhibition when the tablet suspension was utilized, with values similar to those obtained when the live cells, the inactivated cells and the LPS Ogawa were used. No inhibition was observed in the case involving the LPS 0139 (Figure 3). This demonstrates that the antigenic activity of the inactivated cells remains unaffected in the final, tablet formula.
**Example 4**: Immunogenicity of the vaccine
   The vaccine's immunogenicity was studied through the intra-duodenal inoculation of rabbits with the problem samples. The seric antibody kinetics was evaluated using the ELISA and Vibriocidal techniques. Suspensions containing the tablet formula, and inactivated cells of El Tor and Ogawa strain *V. cholera,* were employed as problem samples.
   A laparatomy was carried out to expose the duodenum and inoculate the duodenal lumen with 5 mL of a re-suspended tablet, with an inactivated culture of the vaccine strain or with a saline solution. The seric kinetics of the antibody was determined using the ELISA and Vibriocidal techniques.
   The ELISA technique for determining the kinetics of IgG anti-lipopolysaccharide antibodies:
   1. Cover the Nunc or Maxisorp 96-hole polystyrene plaques with pure LPS at a concentration of 25 µg/mL. Incubate overnight at 4 °C or for 2 hours at 37 °C.
   2. Wash 3 times with Tween 20 in water at a concentration of 0.05%
   3. Block with 150 uL/hole with a skim milk solution at a concentration of 2% in a PBS, for 1 hour at 37°C.
   4. Wash three times.
   5. Make serial solutions of the problem serum in a PBS Tween 0.05%. Incubate for 2 hours at room temperature.
   6. Wash three times as before
   7. Add the conjugated anti IgG-HRP species diluted in 1% milk in a PBS-T and incubate 1 hour at room temperature.
   8. Wash six times.
   9. Develop with o-phenylenediamine at a concentration of 1mg/mL and H₂O₂ at a concentration of 30% in a citrate buffer of pH 4.5.
   10. Read the absorbance at a wavelength of 492 nm.

   The Vibriocidal technique, to test bactericidal antibodies:
   Flat-bottomed sterile plaques were used.
      1. Add 50 µL of the problem serums in the first holes. Add 25 µL of saline solution in the remaining hoies.
      2. Make serial solutions and throw out the last 25 µL.
      3. Add 25 µL of the complement-bacteria mixture (2.5 ml per plaque) and incubate for 1 hour at 37°C.
      4. Add a BHI culture medium (use 125 µL of Bromcresol purple and 10 ml of glucose for 100 ml of the medium. The 10 ml of glucose must be eliminated from the medium).
      5. Manually homogenize the plaque and incubate for 3 hours at 37°C.
      6. The titer is defined as the highest dilution of the serum that is able to inhibit bacterial growth, which is determined by a lack of change in the color of the medium.

### Preparation of the mixture of complement-bacteria:

1. 2 wedges or plaques of BHI agar are sown with the strain of *V. cholerae* conserved and are left to incubate along with two un-sown wedges, at a temperature of 37°C for anything between 18 and 24 hours.
2. Isolated colonies in the plaques, or a number of them cooked in the wedges, are taken and placed in the pre-heated wedges. These are left to incubate for 4 hours at 37°C.
3. Wash the growth found in the wedges with a sterile saline solution at a pH of 7 and adjust the wash to D.O = 0.9 - 0.95.
4. The inactivated cells adjusted are diluted at a 1:10 ratio
5. The complement-bacteria mixture is prepared, where:
   - The complement (guinea pig or human serum, free of vibriocidal antibodies) is diluted at a 1:5 ratio to the cold sterile saline solution.
   - Equal volumes of inactivated cells and diluted complement are mixed

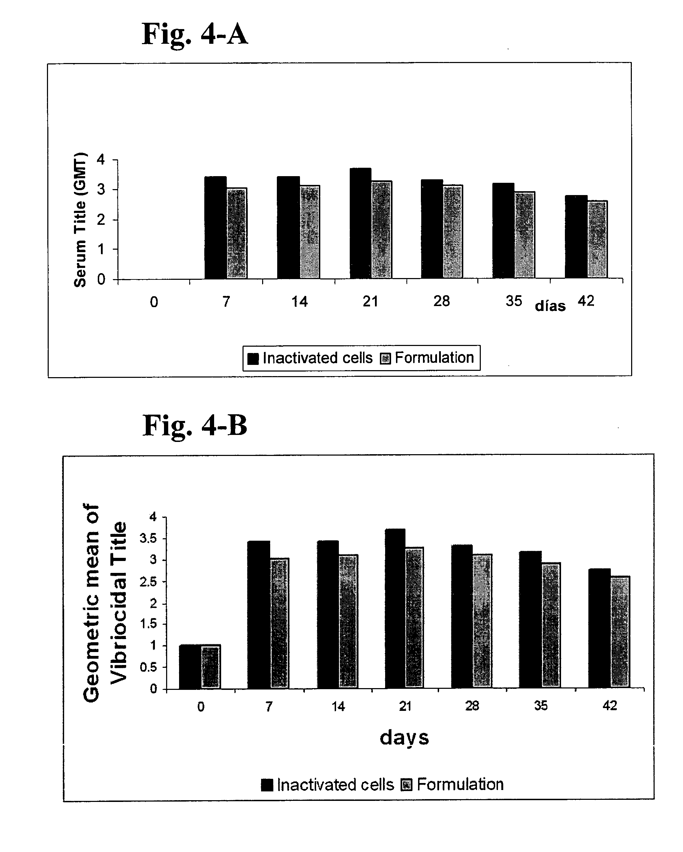

The results present no differences, in the immunogenic capacity to induce anti-lipopolysaccharide antibodies in rabbits, between the tablet formula and the inactivated cells evaluated using the ELISA technique (Figure 4 A), and Vibriocidal antibodies
(Figure 4 B). Examples 1, 2 and 3 demonstrate that the tablet formula described in the present invention is as antigenic and immunogenic in model animals as the inactivated cells themselves, prior to being included in the formula; this is demonstrated both by the presence of antigens in the final tablet formula and by its capacity to induce vibriocidal antibodies.

### Advantages of the proposed solution

The proposed solution is superior to previous vaccine formulas because it is a highly immunogenic product (in its capacity to induce both anti-lipopolysaccharide and vibriocidal antibodies) that can be easily administered, involving none of the potential risks associated with live, genetically manipulated vaccines, nor the difficulties involved in administering the product presented by previous inactivated vaccines.

### Brief description of figures

**Figure 1**. Study of possible interferences of tablet excipients, using the ELISA technique to evaluate the LPS of Vibrio cholerae. From C01 to C09: mixture of excipients with inactivated cells. C10 to C18: excipients alone. C19: inactivated cells alone.
   Excipients used in each of the samples: C01 and C10: starch; C02 and C11: sodium carboxymethylstarch; C03 and C12: croscaramelose; C04 and C13: povidone; C05 and C14: talc; C06 and C15: magnesium stearate; C07 and C16 titanium dioxide; C08 and C17 silicon dioxide; C09 and C18 starch 1500; C19: inactivated cells.
**Figure 2**. Determining possible effects of the excipients on the antigenicity of the vaccine's active complex. The graphs represent the percentages of the average ELISA test result values for samples incubated for 30 days, with respect to the ELISA test result values for the same samples evaluated at time 0. Incubated at 4°C (Figure 2 A) and Incubated at a temperature between 26 and 28°C (Figure 2 B). The excipients evaluated in this example were:
   1.- Lactose, 2.- Starch, 3.- Sodium carboxymethylstarch, 4.- Croscaramelose, 5.-Povidone, 6.- Talc, 7.- Magnesium stearate, 8.- Titanium dioxide, 9.- Inactivated cells.
**Figure 3**. Determining the antigenicity of the lipopolysaccharide (LPS) of Vibrio cholerae in the final tablet formula through an ELISA Monoclonal Antibody Anti-LPS inhibition test.
**Figure 4**. Serum titers of rabbits inoculated with inactivated cells or with the tablet formula of the vaccine. The anti-lipopolysaccharide titles of V. cholerae O1, Ogawa, detected through the ELISA, are shown in Figure 4 A. The vibriocidal titles are shown in Figure 4 B.

## Claims

1. A vaccine composition for cholera composed of:
a. Inactivated cells of *Vibrio cholera*
b. Agglutinants
c. Lubricants
d. Coating substance
e. Filling substance
f. Disintegrating substance

2. The vaccine composition described in Claim 1, with inactivated cells consisting of attenuated strains of *Vibrio cholerae.*

3. The vaccine composition described in Claim 2, with inactivated cells belonging to the serum group 0139.

4. The vaccine composition described in Claim 3, with inactivated cells belonging to the serum group O1.

5. The vaccine composition described in Claim 4, with El Tor or Classic type cells.

6. The vaccine composition described in Claim 5, the inactivated cells belonging to the Ogawa or Inaba serum type.

7. The vaccine composition described in Claim 2, the inactivated cells consisting of wild strains of *Vibrio cholerae.*

8. The vaccine composition described in Claim 7, the inactivated cells belonging to the serum group 0139.

9. The vaccine composition described in Claim 8, the inactivated cells belonging to the serum group O1.

10. The vaccine composition described in Claim 9, with El Tor or Classic bio-type cells.

11. The vaccine composition described in Claim 10, with Ogawa or Inaba serum type cells

12. The vaccine composition described in Claims 1 to 11, containing between 5 x 10⁹ and 10¹¹ cells per tablet.

13. The vaccine composition described in Claim 1, with povidone, gelatin, or carboxymethylcellulose as an agglutinant.

14. The vaccine composition described in Claim 13, with the agglutinants found at a concentration between 1 and 5% of the tablet's total mass.

15. The vaccine composition described in Claim 1, with sodium carboxymethylstarch, magnesium stearate, silicon dioxide, or talc as a lubricants

16. The vaccine composition described in Claim 1, the lubricants found at a concentration between 0.25 and 1.5% of the tablet's total mass.

17. The vaccine composition described in Claim 1, with cellulose acetophtalate, cellulose diethylphthalate, lacquer at 10% or titanium dioxide as a coating substance.

18. The vaccine composition described in Claim 17, with the coating substance found at a concentration between 1 and 2% of the tablet's total mass.

19. The vaccine composition described in Claim 1, with lactose or cornstarch as filling substance.

20. The vaccine composition described in Claim 19, the filling substance found at a concentration between 65 and 80% that of tablet's total mass.

21. The vaccine composition described in Claim 1, with sodium croscaramelose, cornstarch or micro-crystalline cellulose as a disintegrating substance.

22. The vaccine composition described in Claim 21, the disintegrating substance found at a concentration between 1 and 6% that of the tablet's total mass.
